# EUROPÄISCHE PATENTANMELDUNG

(11) **EP 2 301 330 A1**
(43) Veröffentlichungstag der Anmeldung: **30.03.2011**
(21) Anmeldenummer: 10009309.5
(22) Anmeldetag: 08.09.2010
(51) Int. Cl.: A01K 11/00, A61B 5/117, A61B 19/00, A61C 13/00

(54) **Kodierung für ein Gebisselement**

(30) Priorität: 29.09.2009 DE 102009043281
(71) Anmelder: Ditzel GmbH & co. KG Grundstücks- und Beteiligungsgesellschaft, 61137 Schöneck (DE)
(72) Erfinder: Ditzel, Thomas, 61130 Nidderau (DE); Neumeier, Peter, 80807 München (DE)
(74) Vertreter: Knoblauch, Andreas

(57) **Zusammenfassung**

Es wird ein Gebisselement angegeben.
Man möchte eine einfache forensische Identifizierungsmöglichkeit für eine Person oder ein Tier bereitstellen.
Hierzu ist vorgesehen, dass eine Kodierung (4) an dem Gebisselement oder Zahn angeordnet ist. Die Kodierung (4) kann als maschinenlesbarer Barkode (6) in Form einer feuerfesten Nanostruktur (7) auf einem Zahn (2), eine Plombe (3) oder ein Inlet (3) aufgedruckt werden.

## Beschreibung

Die Erfindung betrifft ein Gebisselement, insbesondere ein künstliches Gebisselement.

Ferner betrifft die Erfindung ein Verfahren zum Aufbringen einer Kodierung.

Aus dem Übersichtsreferat "Identifikation unbekannter Toter durch odontologische Untersuchungen" '(Zeitschrift Rechtsmedizin, Vol. 11, S. 37-41, 2001; Springer Berlin Heidelberg) ist ein Verfahren bekannt, unbekannte Tote anhand von Untersuchungen des Zahnsystems zu identifizieren. Ein solches Verfahren wird vor allem dann durchgeführt, wenn andere Identifikationsmerkmale (etwa Personalpapiere oder Fingerabdrücke) in nicht ausreichender Weise vorhanden sind. Zähne weisen Zahnmerkmale auf, mit deren Hilfe sich Personen eindeutig identifizieren lassen. Zudem sind Zähne äußerst robust. Der Zahnschmelz gilt als härteste Substanz des menschlichen Körpers. Man kann bei der Identifikation ausnutzen, dass Zähne im Rahmen zahnärztlicher Behandlungen in zivilisierten Regionen registriert und dokumentiert sind. Bei einer vollständigen Dokumentation kann die Identifikation anhand prä- und postmortaler Befunde erfolgen. Alternativ kann ein Vergleich von prä- und postmortalen Röntgenbildern zur Identifikation führen. Insbesondere Panoramaschichtaufnahmen ermöglichen dem Rechtsmediziner eine zuverlässige Identifikation.

Solche Verfahren haben sich zwar prinzipiell bewährt. Insbesondere bei Verbrennungen stellen die Zähne oft die einzig verbliebene Identifizierungsmöglichkeit dar. In jedem Fall erfordert aber eine solche Identifikation einen erheblichen Dokumentationsaufwand beim behandelnden Zahnarzt. Erschwert wird diese Dokumentation auch dadurch, dass viele Patienten beispielsweise aufgrund eines Ortswechsels oft den Zahnarzt wechseln. Röntgenbilder werden von Teilen der Bevölkerung aufgrund der Strahlenbelastung nur für den Fall einer absoluten Notwendigkeit akzeptiert.

Der Erfindung liegt die Aufgabe zugrunde, eine einfache Identifizierungsmöglichkeit für eine Person oder ein Tier bereitzustellen.

Diese Aufgabe wird bei einem Gebisselement der eingangs genannten Art dadurch gelöst, dass eine Kodierung an dem Gebisselement angeordnet ist.

Mit dieser Ausgestaltung kann man auf einfache Weise Informationen am Gebisselement anbringen. Das Auslesen einer solchen Kodierung ist gegenüber einer Untersuchung am Zahnsystem erheblich einfacher und schneller. Man braucht nicht mehr den behandelnden Zahnarzt oder die behandelnden Zahnärzte ausfindig zu machen, sondern liest einfach die Kodierung vor Ort aus. Eine Strahlenbelastung entsteht dabei auch nicht. Auf diese Weise können schnell medizinische Maßnahmen ergriffen werden und/oder Angehörige informiert werden. Das Auslesen der Kodierung erfolgt über eine Ausleseeinrichtung wie einen Scanner oder eine Kamera.

Als Gebisselement sind Zähne oder Teile des Kieferknochens der Person oder des Tieres denkbar. Es ist aber auch möglich, die Kodierung an dem künstlichen Gebisselement anzubringen. Insbesondere ist es möglich, die Kodierung an einer Plombe, einer Brücke, einem Inlet, einem Implantat, einem künstlichen Gebiss oder ganz allgemein an einem Zahnersatzteil anzubringen. Die Anordnung der Kodierung an dem künstlichen Gebisselement ist dabei besonders vorteilhaft. Sie kann außerhalb eines Körpers der Person oder des Tieres angebracht werden. Man muss also keine Rücksicht beim Anbringen auf den Körper nehmen.

Insbesondere eine individuelle personenbezogene oder tierbezogene Kodierung ist denkbar. Diese kann Informationen zu Geburtsort, Wohnort, Name, Blutgruppe, Krankheiten, benötigter Medizin oder andere biometrische Daten der Person oder des Tieres beinhalten. Auch kann gespeichert werden, wer im Fall eines Unfalls benachrichtigt werden soll.

Vorzugsweise ist die Kodierung als Farbauftrag ausgebildet. Dieser Farbauftrag kann dabei eine individuelle personenbezogene Farbgebung aufweisen. Die Farbgebung kann dabei so ausgestaltet sein, dass man die Farbgebung eindeutig der zu identifizierenden Person, ähnlich einem Fingerabdruck, zuweisen kann. Der Farbauftrag wird dabei unlöslich am Gebisselement angeordnet.

Vorzugsweise weist die Kodierung eine optoelektronisch lesbare Schrift auf. Bei der optoelektronisch lesbaren Schrift kann es sich um einen Strichkode, Balkenkode oder Barkode handeln. Die Informationen können also in binären Symbolen gespeichert sein. Es sind ein- oder mehrdimensionale Kodierungen denkbar.

Bevorzugterweise ist die Kodierung als Nanostruktur ausgebildet. Die Kodierung kann so klein sein, dass sie einerseits für ein menschliches Auge nicht sichtbar ist. Andererseits ist es aber für die Ausleseeinrichtung ohne Weiteres möglich, die Kodierung auszulesen. Der Mensch, der das kodierte Gebisselement trägt, hat also keinen ästhetischen Nachteil. Die Gefahr, dass in der Kodierung enthaltene Informationen an Unbefugte weitergegeben werden, ist damit auch reduziert.

Bevorzugterweise ist ein Kodierungselement der Kodierung in einem vorbestimmten Wellenlängenintervall lesbar. Das Wellenlängenintervall kann in einem nicht sichtbaren Teil des Lichts sein. Dies hat den Vorteil, dass Unbefugte nicht ohne technischen Aufwand die Kodierung auslesen können.

Bevorzugterweise sind Kodierungselemente der Kodierung jeweils in voneinander verschieden vorbestimmten Wellenlängenintervallen lesbar. Ein Teil der Kodierung kann also in einem bestimmten Wellenlängenintervall enthalten sein, während ein anderer Teil der Kodierung Teil eines anderen Wellenlängenintervalls ist. Die Wellenlängenintervalle können sich dabei überlappen, sie können aber auch disjunkt sein. Mit optischen Filtern und wellenlängensensiblen Empfangselementen kann man dann die in den jeweiligen Kodierungselementen enthaltenen Informationen auslesen und sie danach zusammenführen. Dazu kann eine Datenverarbeitungseinrichtung vorgesehen sein. Man erhält auf diese Weise auch eine hohe Informationsdichte, da man mehrere Wellenlängenintervalle, d.h. Kanäle, gleichzeitig benutzt.

Vorzugsweise ist die Kodierung nur mit einem Dekodierer entschlüsselbar. Unbefugte können also die Kodierung nicht lesen. Der Dekodierer kann auch eine zusätzliche Datenverarbeitungseinrichtung aufweisen. Der Dekodierer benutzt dann die Informationen der Kodierung und dekodiert sie mit Hilfe der Datenverarbeitungseinrichtung.

Vorzugsweise weist die Kodierung einen Hitzeschutz auf. Insbesondere bei Verbrennungen ist so gesichert, dass die Kodierung weiterhin lesbar bleibt und unbeschädigt ist. Der Hitzeschutz kann als Beschichtung ausgebildet sein. Es ist aber auch möglich, dass man der Kodierung eine Beimischung zufügt, die die Kodierung hitzebeständig macht.

Die Aufgabe wird bei einem Verfahren der eingangs genannten Art dadurch gelöst, dass man die Kodierung an einem Gebisselement, insbesondere an einem künstlichen Gebisselement, aufbringt.

In diesem Fall ist es nicht mehr erforderlich, die Person oder das Tier anhand odontologischer Untersuchungen zu identifizieren. Nachdem man einmal die Kodierung angebracht hat, kann man in der Kodierung gespeicherte Informationen einfach auslesen. Die Kodierung kann biometrische Informationen, aber auch beliebige andere Informationen enthalten, wie bereits erwähnt. Als Gebisselement kann man Teile eines menschlichen oder tierischen Gebisses verwenden. Man kann aber auch künstlich hergestellte Gebisselemente verwenden.

Vorzugsweise trägt man die Kodierung mittels eines Farbauftrags auf. Das Auftragen kann beispielsweise mit einer Vorrichtung erfolgen, wie sie aus dem Patent DE 10 2006 061 893 bekannt ist. Man kann den Farbauftrag so auftragen, dass er der Person oder dem Tier eindeutig zugeordnet werden kann.

Bevorzugterweise versieht man die Kodierung mit einem Hitzeschutz. Den Hitzeschutz kann man aufbringen, nachdem man die Kodierung aufgebracht hat. Dies kann aber auch in einem Schritt erfolgen, in dem man der Kodierung eine hitzebeständige Beimischung zufügt.

Vorzugsweise verschlüsselt man die Kodierung. Unter Verwendung eines oder mehrerer Schlüssel werden lesbare Informationen in Geheiminformationen umgewandelt. Gerade sensible biometrische Daten können nur noch von einem Befugten gelesen werden, der im Besitz des Schlüssels ist.

Hierbei ist von Vorteil, wenn man zum Verschlüsseln der Kodierung verschiedene vorbestimmte Wellenlängenintervalle verwendet. Bestimmte Teile der in der Kodierung gespeicherten Informationen sind nur in bestimmten Wellenlängenintervallen lesbar. Um die Kodierung lesen zu können, benötigt man dann verschiedene optische Filter und/oder wellenlängensensible optoelektrische Empfangselemente. Mit Hilfe einer Datenverarbeitungseinrichtung kann man dann die ausgelesenen Informationen zusammenführen und erhält so die vollständigen in der Kodierung gespeicherten Informationen.

Bevorzugterweise liest man die Kodierung aus und vergleicht in der Kodierung enthaltene Informationen mit weiteren Informationen. Man kann zuerst die in der Kodierung enthaltenen Informationen, die beispielsweise im Farbauftrag enthalten sind, auslesen. Die so gewonnenen Informationen vergleicht man dann mit weiteren Informationen, die beispielsweise in einer Datei gespeichert sind. So kann die das Gebisselement tragende Person identifiziert werden und möglicherweise können noch zusätzliche Informationen über sie gewonnen werden.

Die Erfindung wird im Folgenden anhand eines bevorzugten Ausführungsbeispiels in Verbindung mit den Zeichnungen beschrieben. Hierin zeigen:
- Fig. 1: ein als Zahn ausgebildetes Gebisselement mit mehreren Kodierungen. Ein Teil des Zahns ist durch ein künstliches Gebisselement ersetzt.
- Fig. 2: einen Schnitt durch eine Krone mit Kodierung und einem Hitzeschutz,
- Fig. 3: ein Diagramm mit einer Informationsdichte als Funktion einer Wellenlänge,
- Fig. 4: eine schematische Anordnung einer Ausleseein- richtung, einem Dekodierer, einer Datenverar- beitungseinrichtung und einer Datei.

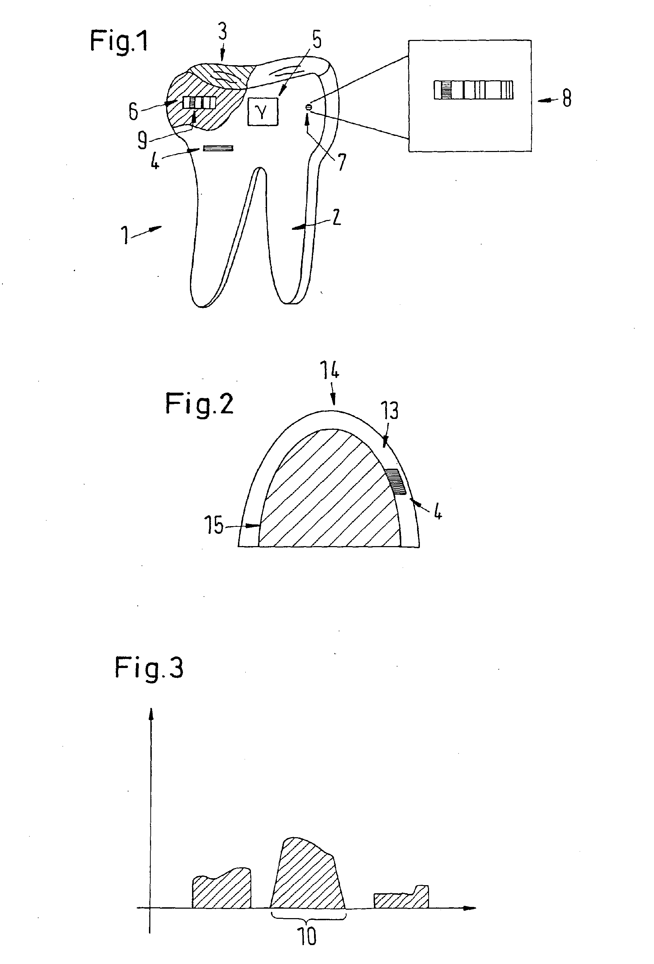

In Fig. 1 ist ein als Zahn ausgebildetes Gebisselement 1 dargestellt. Das Gebisselement 1 besteht aus einem natürlichen Gebisselement 2 und einem künstlichen Gebisselement 3. An dem Gebisselement 1 sind mehrere Kodierungen 4 angeordnet. Die Kodierungen 4 können am natürlichen Gebisselement 2, aber auch am künstlichen Gebisselement 3 angeordnet sein. Beides ist für alle Ausgestaltungen der Kodierungen 4 möglich. Das natürliche Gebisselement 2 kann ein Zahn oder ein Teil des Kieferknochens eines menschlichen Gebisses sein. Das künstliche Gebisselement 2 kann als Plombe, Brücke, Inlet, Implantat, künstliches Gebiss oder als Zahnersatzteil ausgebildet sein.

In der Kodierung können biometrische Informationen einer Person oder eines Tieres gespeichert sein.

Die Kodierung kann dabei jeweils verschiedene Ausgestaltungen haben. Insbesondere ist es denkbar, dass die Kodierung 4 als Farbauftrag 5 ausgebildet ist. Der Farbauftrag 5 kann permanent an das Gebisselement 1 angebracht werden. Insbesondere kann man den Farbauftrag 5 so wählen, dass dessen Farbe im nicht sichtbaren Bereich des Lichts liegt. Dies erschwert einem Unbefugten ein Auslesen der Kodierung 4.

Man kann die Kodierung 4 auch eine optoelektronisch lesbare Schrift 6 aufweisen lassen. Die optoelektronisch lesbare Schrift 6 kann einen Strichkode, einen Balkenkode oder einen Barkode aufweisen. Die Kodierung 4 kann eindimensional, aber auch mehrdimensional sein. Es ist möglich, die Kodierung 4 so zu verwenden, wie sie im Einzelhandel benutzt wird. An sich ist es möglich, die Kodierung 4 so auszugestalten, dass man sie mit bloßem Auge sehen kann. In der Praxis wird man die Kodierung 4 aber so ausgestalten, dass sie mit bloßem Auge nicht lesbar ist. Insbesondere wird man eine Nanostruktur 7 wählen. Die Nanostruktur 7 ist in Fig. 1 auf dem Gebisselement 1 und als Zoom 8 stark vergrößert zu sehen. Die Kodierung 4 fällt so einem Dritten nicht auf, sie ist aber trotzdem lesbar.

Man kann ein Kodierungselement 9, aber auch die gesamte Kodierung 4 in einem vorbestimmten Wellenlängenintervall 10 lesbar ausgestalten. Wählt man als Wellenlängenintervall 10 den nicht sichtbaren Teil des Lichts, dann ist die Kodierung 4 unabhängig von ihrer Größe nicht sichtbar.

Wie in Fig. 3 dargestellt kann man auch Kodierungselemente 9 der Kodierung 4 jeweils in voneinander verschiedenen vorbestimmten Wellenlängenintervallen 10 lesbar ausgestalten. In Fig. 3 ist auf der Y-Achse eine Informationsdichte und auf der X-Achse eine Wellenlänge aufgetragen. Beispielsweise im Wellenlängenintervall 10 ist die Informationsdichte von Null verschieden, d.h. zumindest eines der Kodierungselemente 9 ist in diesem Wellenlängenintervall 10 lesbar. Man kann ein oder auch mehrere Wellenlängenintervalle 10 vorsehen, wobei die Wellenlängenintervalle 10 disjunkt, aber auch überlappend ausgestaltet sein können. Um die gesamte Kodierung 4 auslesen zu können, kann man eine Ausleseeinrichtung 11 vorsehen, die einen optischen Filter und/oder ein wellenlängensensibles Empfangselement aufweist.

Um sicher zu gehen, dass Niemand Unbefugtes die Kodierung 4 lesen kann, wird man sie verschlüsseln. Um die Kodierung 4 wieder zu entschlüsseln, muss ein Dekodierer 12 verwendet werden, ohne den die Kodierung 4 nicht lesbar ist. Zum Verschlüsseln kann man auch verschiedene vorbestimmte Wellenlängenintervalle 10 benutzen.

Idealerweise wird man einen Hitzeschutz 13 vorsehen. Der Hitzeschutz 13 verhindert dann, dass die Kodierung 4 bei Bränden etc. beschädigt wird. Gerade bei Bränden, bei denen Ausweise etc. verbrennen, bleibt die Kodierung 4 weiter auslesbar. In Fig. 2 ist eine Krone 14 zu sehen. An ihrer Oberfläche 15 ist die Kodierung 4 angeordnet. Der Hitzeschutz 13 umgibt Krone 14 und Kodierung 4. Der Hitzeschutz 13 kann zusätzlich auch noch eine mechanische Funktion übernehmen. Er kann aus hartem Material gefertigt sein und so die Beschädigung der Krone 14 mit Kodierung 4 im Alltag, aber auch im Falle einer Explosion verhindern.

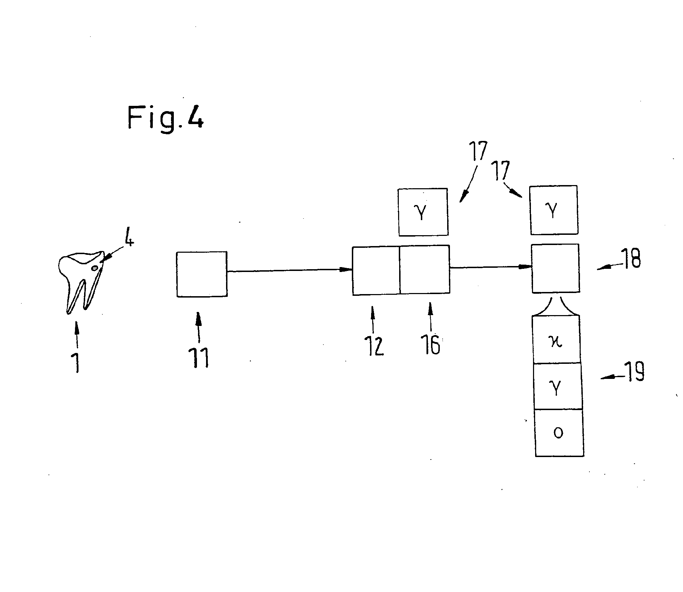

In Fig. 4 ist das Gebisselement 1 mit Kodierung 4 gezeigt. Die Kodierung 4 wird von der Ausleseeinrichtung 11 ausgelesen. Sie kann verschlüsselt sein. Deswegen ist hinter der Ausleseeinrichtung 11 der Dekodierer 12 angeordnet, der die Kodierung 4 entschlüsselt. Dahinter ist eine Datenverarbeitungseinrichtung 16 angeordnet. Die Datenverarbeitungseinrichtung 16 vergleicht dann in der Kodierung 4 enthaltene Informationen 17, mit denen in einer Datei 18 gespeicherten weiteren Informationen 19. So kann beispielsweise aufgrund der Kodierung 4 und dem Vergleich mit den in der Datei 18 enthaltenen weiteren Informationen 19 die Person identifiziert werden. Die Datenverarbeitungseinrichtung 16 kann aber auch ohne Dekodierer 12 und ohne Datei 18 benutzt werden. Dann kann sie beispielsweise dazu dienen, mehrere ausgelesene Kodierungselemente 9 zur gesamten Kodierung 4 zusammenzufassen.

## Patentansprüche

1. Gebisselement, insbesondere künstliches Gebisselement, **dadurch gekennzeichnet, dass** eine Kodierung (4) an dem Gebisselement (1) angeordnet ist.

2. Gebisselement nach Anspruch 1, **dadurch gekennzeichnet, dass** die Kodierung (4) als Farbauftrag (5) ausgebildet ist.

3. Gebisselement nach einem der Ansprüche 1 oder 2, **dadurch gekennzeichnet, dass** die Kodierung (4) eine optoelektronisch lesbare Schrift (6) aufweist.

4. Gebisselement nach einem der Ansprüche 1 bis 3, **dadurch gekennzeichnet, dass** die Kodierung (4) als Nanostruktur (7) ausgebildet ist.

5. Gebisselement nach einem der Ansprüche 1 bis 4, **dadurch gekennzeichnet, dass** ein Kodierungselement (9) der Kodierung (4) in einem vorbestimmten Wellenlängenintervall (10) lesbar ist.

6. Gebisselement nach Anspruch 5, **dadurch gekennzeichnet, dass** Kodierungselemente (9) der Kodierung (4) jeweils in voneinander verschiedenen vorbestimmten Wellenlängenintervallen (10) lesbar sind.

7. Gebisselement nach einem der Ansprüche 1 bis 6, **dadurch gekennzeichnet, dass** die Kodierung (4) nur mit einem Dekodierer (12) entschlüsselbar ist.

8. Gebisselement nach einem der Ansprüche 1 bis 7, **dadurch gekennzeichnet, dass** die Kodierung (4) einen Hitzeschutz (13) aufweist.

9. Verfahren zum Aufbringen einer Kodierung, **dadurch gekennzeichnet, dass** man die Kodierung an einem Gebisselement, insbesondere an einem künstlichen Gebisselement, aufbringt.

10. Verfahren nach Anspruch 9, **dadurch gekennzeichnet, dass** man die Kodierung mittels eines Farbauftrags aufträgt.

11. Verfahren nach einem der Ansprüche 9 oder 10, **dadurch gekennzeichnet, dass** man die Kodierung mit einem Hitzeschutz versieht.

12. Verfahren nach einem der Ansprüche 9 bis 11, **dadurch gekennzeichnet, dass** man die Kodierung verschlüsselt.

13. Verfahren nach Anspruch 12, **dadurch gekennzeichnet, dass** man zum Verschlüsseln der Kodierung verschiedene vorbestimmte Wellenlängenintervalle verwendet.

14. Verfahren nach einem der Ansprüche 9 bis 13, **dadurch gekennzeichnet, dass** man die Kodierung ausliest und die in der Kodierung enthaltenen Informationen mit weiteren Informationen vergleicht.
